Europäisches Patentamt

European Patent Office

Office européen des brevets

(19)

(11) Veröffentlichungsnummer: **0 144 387**

**B1**

(12) EUROPÄISCHE PATENTSCHRIFT

(45) Veröffentlichungstag der Patentschrifft:
28.02.90

(21) Anmeldenummer: 84902203.3

(22) Anmeldetag: 25.05.84

(86) Internationale Anmeldenummer:
PCT/EP 84/00160

(87) Internationale Veröffentlichungsnummer:
WO 84/04920 (20.12.84 Gazette 84/30)

(51) Int. Cl. 5: **C 07 D 301/16**, **C 07 D 303/04**,
**C 07 D 303/14**, **C 07 D 303/42**

(54) KONTINUIERLICHE, KATALYTISCHE EPOXIDATION VON OLEFINISCHEN DOPPELBINDUNGEN MIT WASSERSTOFFPEROXID UND AMEISENSÄURE.

(30) Priorität: 03.06.83 DE 3320219

(43) Veröffentlichungstag der Anmeldung:
19.06.85 Patentblatt 85/25

(45) Bekanntmachung des Hinweises auf die Patenterteilung:
28.02.90 Patentblatt 90/09

(84) Bennante Vertragsstaaten:
AT BE CH DE FR GB LI LU NL SE

(56) Entgegenhaltungen:
FR-A-1 140 486
US-A-3 458 536

Die Akte enthält technische Angaben, die nach dem Eingang der Anmeldung eingereicht wurden und die nicht in dieser Patentschrift enthalten sind.

(73) Patentinhaber: Henkel Kommanditgesellschaft auf Aktien
Postfach 1100 Henkelstrasse 67
D-4000 Düsseldorf-Holthausen (DE)

(72) Erfinder: DIECKELMANN, Gerhard
Luisenstrasse 19
D-4010 Hilden (DE)
Erfinder: ECKWERT, Klemens
Heinrich-Lerschweg 54
D-4000 Düsseldorf (DE)
Erfinder: JEROMIN, Lutz
Am Bandsbusch 88
D-4010 Hilden (DE)
Erfinder: PEUKERT, Eberhard
Dürerweg 15
D-4010 Hilden (DE)
Erfinder: STEINBERNER, Udo
Gerhard-Hauptmann Strasse 44
D-4006 Erkrath 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die Erfindung betrifft ein mehrstufiges Verfahren zur kontinuierlichen Epoxidation von Doppelbindungen end und innenständiger Olefine mit mehr als 12 C-Atomen, ungesättigter Fettsäuren und deren Ester sowie ungesättigter Alkohole mit 8 bis 18, vorzugsweise 18,C-Atomen im Alkylrest und Triglyceriden, vorzugsweise Sojaöl, mit Wasserstoffperoxid und Ameisensäure in Gegenwart eines Katalysators.

Die Epoxidierung ungesättigter Fettsäurederivate, in erster Linie von Sojabohnenöl, wird in großem Maßstab betrieben. Man erhält dadurch mit Polyvinylchlorid (PVC) verträgliche Weichmacher, die gleichzeitig als Stabilisatoren, hauptsächlich gegen Wärmebeanspruchung, wirken (Ullmann's Encyclopädie der Technischen Chemie, Verlag Chemie Weinheim (1975), Bd. 10, 574). Nach Gutachten des Bundesgesundheitsamtes und der Food and Drug Administration ist epoxidiertes Sojabohnenöl auch als Additiv für Kunststoffe zugelassen, die Kontakt mit Lebensmitteln haben.

Als Epoxidierungsmittel wird heute Perameisensäure bevorzugt, die sich in situ aus Ameisensäure und Wasserstoffperoxid generieren lässt. Die Reaktion läuft in einem heterogenen Gemisch aus persäurehaltiger wässriger Phase und Ölphase bei leicht erhöhter Temperatur (50 – 80°C) ab.

Die FR-A-1 140 486 beschreibt ein mehrstufiges Epoxidationsverfahren bei dem man das zu epoxidierende Substrat in die erste Reaktionsstufe einbringt und im einfachen Gegenstromprinzip der organischen Persäure entgegenführt, die in der letzten Reaktionsstufe aus einer Carbonsäure und einem Peroxid hergestellt wird.

Es ist bekannt, daß die Durchführung chemischer Prozesse in einer kontinuierlichen Rührkesselkaskade gegenüber dem diskontinuierlichen Rührkesselverfahren aufgrund des Verweilzeitspektrums schlechtere Umsätze und Ausbeuten liefert, da ein entsprechender Umsatz nur nach einer längeren mittleren Verweilzeit oder durch hohe Überschüsse eines oder mehrerer Reaktionspartner erzielt werden kann. Im Verfahren der Epoxidation von Fettsäurederivaten kann die Verweilzeit nicht beliebig erhöht werden, ohne daß die Epoxidausbeute darunter leidet, da bei der Epoxidation unerwünschte Epoxidfolgeprodukte durch Ringöffnungsreaktionen entstehen, die im wesentlichen durch die eingesetzte Ameisensäure initiiert werden. Aus dem gleichen Grunde kann auch die Konzentration an Ameisensäure nicht über einen bestimmten Wert gesteigert werden.

Außerdem ist es aus Gründen der Arbeitssicherheit notwendig, die Konzentration an Perameisensäure möglichst niedrig zu halten, da diese stark oxidierende Säure beim Erhitzen oder bei Kontakt mit Metallen wie z. B. Eisen oder Kupfer oder stark reduzierenden Verbindungen explosiv abreagieren kann. Als einzige Möglichkeit zur Erzielung hoher Ausbeuten im kontinuierlichen Verfahren in der Rührkeßselkaskade bleibt die Erhöhung des $H_2O_2$ – Überschusses gegenüber dem diskontinuierlichen Verfahren.

Die Qualität des olefinischen Ausgangsmaterials und der entsprechenden Epoxide wird im wesentlichen durch zwei Kennzahlen erfasst, nämlich die Iodzahl (IZ) und (den Epoxidwert (EPO). Während die Isodzahl ein Maß für die Anzahl der ungesättigten Doppelbindungen ist, gibt der Epoxidwert den prozentualen Anteil (Gewichtsprozent) Epoxidsauerstoff an; er ist somit ein Maß für die Epoxidausbeute. Die Qualität des Epoxids ist (umso besser, je höher der Epoxidwert und je niedriger die Iodzahl liegen.

Beispielsweise ist ein Sojaölepoxid sehr guter Qualität durch IZ ≤ 2,5 und EPO ≥ 6,5 charakterisiert, bei guter Qualität werden Werte von IZ ≤ 5,0 und EPO ≥ 6,3 erreicht. Sojaölepoxid mit einer Iodzahl über 5,0 oder einem Epoxidwert unter 6,3 wird als anwendungstechnisch minderwertig betrachtet. Das Ziel eines Epoxidationsverfahrens muß es also sein, Epoxid herzustellen, das sowohl eine niedrige Iodzahl als auch einen hoben Epoxidwert aufweist.

Während eine niedrige Iodzahl, die einem hohen Umsatz entspricht, auch bei geringem Überschuss an Wasserstoffperoxid stets durch eine entsprechend lange Verweilzeit erzielt werden kann, gibt es für die maximale Epoxidausbeute eine optimale Verweilzeit. Wird diese überschritten, so fällt der Epoxidwert wieder ab.

Beim Kaskadenverfahren sowie bei der diskontinuierlichen Epoxidation nimmt die Dichte der schwereren wässrigen Phase, im folgenden auch als "Sauerwasser" bezeichnet, mit fortschreitender Reaktion ab, während die Dichte der öligen Phase zunimmt. Der Dichteunterschied zwischen den Phasen wird sehr klein, was eine Trennung der Phasen durch Schwerkraftabscheidung erschwert. Außerdem ruft die bei der Epoxidation entstehende Wärme im Abscheider infolge von Temperaturgradienten Konvektionsströmungen hervor, die von aufsteigenden Gasblasen, die sich bei der Zersetzung der wässrigen Phase bilden, noch verstärkt werden.

Bei dem erfindungsgemäßen Verfahren, im folgenden als "Kreuzgegenstromverfahren" bezeichnet, ist es trotzdem gelungen, eine gute Phasentrennung durch Schwerkraftabscheidung zu erreichen. Es werden in jeder Reaktionsstufe Konzentrationen verwirklicht, die eine zur Phasentrennung hinreichend große Dichtedifferenz zwischen wässriger und öliger Phase gewährleisten. Die Abscheidung wird weiterhin begünstigt durch Füllkörper aus Glas oder Keramik.

Die Erfindung betrifft demgemäß ein mehrstufiges Verfahren zur kontinuierlichen Epoxidation von Doppelbindungen end- und innenständiger Olefine mit mehr als 12 C-Atomen, ungesättigter Fettsäuren und deren Ester sowie ungesättigter Alkohole mit 8 bis 18, vorzugsweise 18 C-Atomen im Alkylrest und Triglyceriden, vorzugsweise Sojaöl, unter Verwendung von in situ aus Wasserstoffperoxid und Ameisensäure gebildeter Perameisensäure, das dadurch gekennzeichnet ist, daß man die Reaktanden Olefin und Wasserstoffperoxid/Ameisensäure in einer n-stufigen mindestens 3stufigen, vorzugsweise 4stufigen, Reaktionskaskade, deren einzelne Stufen aus je einem Rührkesselreaktor und einem Phasenabscheider bestehen, bei Umgebungsdruck im Kreuzgegenstrom führt, wobei die Olefinphase in die erste Reaktionsstufe, das Wasserstoffperoxid und die Ameisensäure in getrennten Strömen der vorletzten Reaktionsstufe zudosiert und die Olefin- und die Wasserstoffperoxid/Ameisensäure-Phase (Sauerwasserphase) in jeder Reaktionsstufe nach Austritt aus dem Reaktor in einem Phasenabscheider voneinander getrennt werden, wobei die Olefinphase nacheinander die Reaktionsstufen I bis n durchläuft, während das Sauerwasser nach der Reaktionsstufe (n-1) die Reaktionsstufen in der Reihenfolge (n-2) bis II im Gegenstrom zur Olefinphase und anschließend die Stufen n und I durchläuft und die Reaktionstemperatur in den Reaktoren 50 - 80°C, bevorzugt 60 - 70°C, in den Phasenabscheidern jedoch in der wässrigen Phase 15 bis 40°C und in der olefinhaltigen Phase 30 bis 60°C beträgt.

Wird eine Reaktionsstufenzahl mit n ≠ IV gewählt, so gilt (für die Führung der Phasen mit den Reaktanden: Die olefinische Phase durchläuft die Anzahl der Reaktionsstufen immer in steigender Reihenfolge von I bis n. Für die Reaktionspartner Wasserstoffperoxid und Ameisensäure liegt die Hauptzugabestelle im Reaktor der Stufe (n-1). Nach dem Durchlauf bis zur Reaktionsstufe II im Gegenstrom zur olefinischen Phase wird die Sauerwasserphase über die n-te Stufe zur Reaktionsstufe 5 geführt. Eine je nach Art der olefinischen Phase erforderliche Aufteilung des Wasserstoffperoxid und/oder des Ameisensäurestroms erfolgt auf Reaktoren der Stufen 55 bis (n-1). Ein der n-ten Stufe nachgeschalteter Nachreaktionsbehälter wird hierbei nicht als eigenständige Reaktionsstufe gezählt, da zu dieser Einheit kein Phasenabscheider gehört.

Um eine gute Phasentrennung zu erreichen, ist es wichtig, daß die Reaktion im Phasenabscheider sofort gestoppt und die Zersetzung der bereits abgeschiedenen wässrigen Phase unterbunden wird. Aus diesem Grund besitzt jeder Abscheider zwei eingebaute, voneinander unabhängig betriebene Wärmetauscher zur Kühlung der olefinhaltigen und der wässrigen Phase. Die wässrige Phase wird möglichst kalt gehalten, um Zersetzungsverluste an Wasserstoffperoxid und Ameisensäure zu vermeiden und die Abscheidung der wässrigen Phase aus der (olefinhaltigen Phase nicht durch aufsteigende $CO_2$-Bläschen, die bei der Zersetzung der Perameisensäure entstehen, zu beeinträchtigen. Die wäßrige Phase wird dazu auf eine Temperatur von 15 - 40°C, bevorzugt 20°C, gekühlt.

Da die Dichtedifferenz zwischen olefinhaltiger und wässriger Phase mit fallender Temperatur abnimmt, darf das Gemisch aus homogener olefinhaltiger Phase und disperser wässriger Phase im Abscheider nicht zu stark gekühlt werden. Die Temperaturen in der olefinhaltigen Phase liegen deshalb zwischen 30 und 60°C und bis zu 30°C niedriger als die Reaktortemperatur der entsprechenden Stufe. Dadurch wird auch ein unkontrolliertes Nachreagieren, das je nach Konzentrationsverhältnissen bei 50 - 70°C einsetzt, verhindert.

Das aus dem Reaktor austretende heiße Reaktionsgemisch wird unterhalb der Phasengrenze dem Abscheider zugeführt. Es steigt aufgrund der Dichtedifferenz nach oben und durchströmt die kalte wässrige Phase, wobei es sich abkühlt und die Reaktion gestoppt wird. Außerdem wird beim Durchströmen der wässrigen Phase bereits ein Teil des Sauerwassers aus dem Reaktionsgemisch abgeschieden.

Durch die Trennung der Phasen wird beim Kreuzgegenstromverfahren eine Anlagenschaltung ermöglicht, mit der, im Vergleich zu dem diskontinuierlichen Verfahren, bei gleichem Überschuss an $H_2O_2$ höhere Epoxidausbeuten bzw. gleiche Expoxidausbeuten bei geringerem Überschuss an $H_2O_2$ erzielt werden. Die Zugabe der Reaktionspartner erfolgt derart, daß auch eine unvollkommene Trennung der Phasen infolge der Löslichkeit von Wasserstoffperoxid und Ameisensäure in der olefinhaltigen Phase nicht zu Sauerwasserverlusten führt.

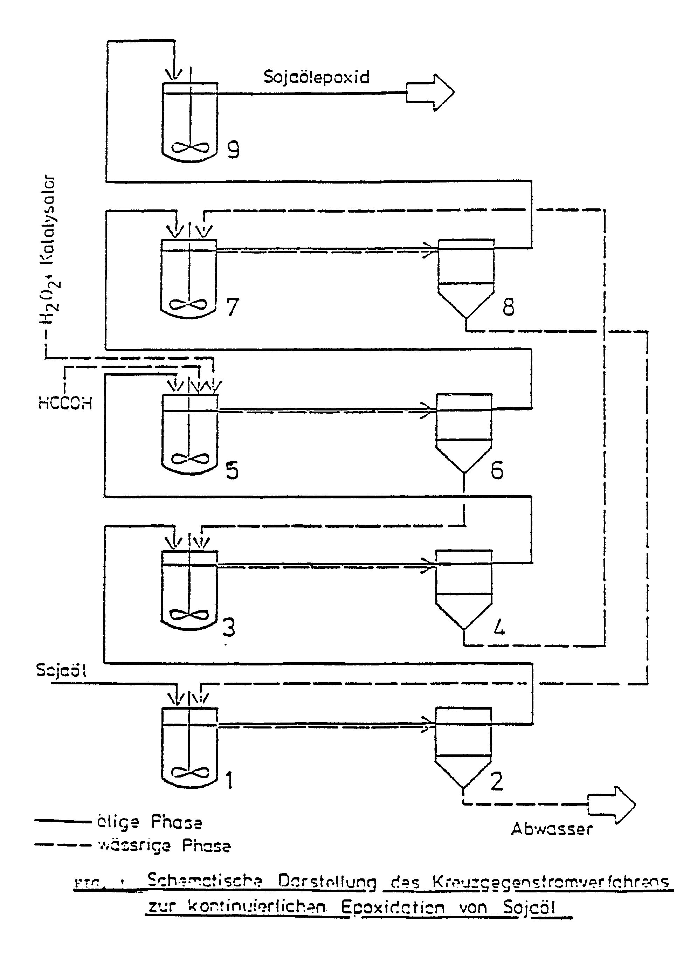

Die kontinuierliche Umsetzung von olefinische Doppelbindungen enthaltenden Verbindungen zu den entsprechenden Epoxiden erfolgt in einer mehrstufigen Anlage, die bei Umgebungsdruck nach dem Mischer-Ascheider-Prinzip arbeitet und deren Fließschema in Fig. 1 dargestellt ist.

Jede Stufe besitzt einen Rührbehälter als Reaktor 1, 3, 5, 7 und 9, in dem die Reaktion in flüssiger Phase stattfindet. Das aus dem Reaktor austretende Gemisch aus teilweise epoxidiertem Olefin, Wasserstoffperoxid, Ameisensäure, Wasser und Katalysator, wird im Abschnider 2, 4, 6 und 8 getrennt in eine wässrige Phase, bestehend aus Wasserstoffperoxid, Ameisensäure, Wasser und Katalysator, sowie eine olefinische Phase, bestehend aus entsprechend der jeweiligen Reaktionsstufe epoxidiertem Olefin und gelösten oder dispergierten Anteilen der wässrigen Phase. Die olefinische und die wässrige Phase werden anschließend den Reaktoren voneinander verschiedener Stufen zugeführt (durchgezogene bzw. unterbrochene Linien).

Die Zugabe des olefinische Doppelbindungen enthaltenden Reaktionspartners erfolgt im Reaktor 1. Anschliessend durchläuft er die Abscheider und Reaktoren in der Reihenfolge aufsteigender Zahlen von 2 bis 9. Im Reaktor 5 werden 70 %-iges Wasserstoffperoxid zusammen mit dem Katalysator und 85 %-iger Ameisensäure zudosiert. Die wässrige Phase durchläuft die Reaktoren und Abscheider in der Reihenfolge 5 – 6 – 3 – 4 – 7 – 8 – 1 – 2. Das im Abscheider 6 in der Olefinphase gelöste und mitgeschleppte hochkonzentrierte Sauerwasser wird im Reaktor 7 mit der niedrig konzentrierten wässrigen Phase aus dem Abscheider 4 ausgewaschen und anschliessend nach Abtrennung von der olefinhaltigen Phase im Abscheider 8 dem Reaktor 1 zugeführt. Die olefinische Phase aus dem Abscheider 8 gelangt zusammen mit dem nicht abgetrennten Sauerwasser in den Nachreaktionsbehälter 9, wo das mitgeschleppte Wasserstoffperoxid noch weiter abgebaut wird.

Die Temperaturen in den Reaktoren werden durch indirekte Kühlung konstant gehalten und betragen 50 bis 80°C, bevorzugt 70°C, in den Reaktoren 1, 3, 5 und 7; im Nachreaktionsbehälter 9 beträgt die Temperatur 40 bis 60°C, bevorzugt 50°C. Die mittlere Verweilzeit pro Reaktor beträgt 1,5 bis 2 h in den Reaktoren 3, 3, 5 und 7 und 3 - 8 h im Nachreaktionsbehälter 9.

Die Abscheider haben die gleichen Nutzvolumina wie die Reaktoren der entsprechenden Stufen. Die Verweilzeit der wässrigen Phase soll so gering sein, die es aus konstruktiven Gründen möglich ist, um Zersetzungsver luste zu vermeiden und instationäre Betriebsschwankungen, wie sie beispielsweise beim Anfahren der Anlage auftreten, schnell ausgleichen zu können.

Durch die Kühlung der Abscheider liegen die Temperaturen der den Reaktoren zugeführten Mengenströme niedriger als die entsprechenden Reaktionstemperaturen, d. h. die Reaktoren werden teilweise durch die Einsatzmengenströme gekühlt und dadurch die Kühlsysteme der Reaktoren entlastet.

Beim Kreuzgegenstromverfahren ist ein völliger Verzicht auf den Einsatz eines Katalysators möglich. Es können aber zur Verkürzung der Verweilzeit die für die in-situ-Epoxidation bekannten Katalysatoren, wie Schwefelsäure oder Phosphorsäure, eingesetzt werden. Besonders vorteilhaft ist die Verwendung von Hydroxyethan 1,2-diphosphonsäure (HEDP), da diese Säure nicht nur katalytisch, sondern auch stabilisierend auf $H_2O_2$ wirkt, so daß nur geringe Zersetzungsverluste an Wasserstoffperoxid auftreten.

Die Erfindung wird durch die nachstehenden Beispiele näher erläutert.

## Beispiel 1

In einer vierstufigen Mixer-Settler-Anlage wurde kontinuierlich 40 kg/h Sojaöl mit einer Jodzahl von 130, was einem theoretisch erreichbaren Epoxidwert (Epoxidsauerstoffgehalt) von 7,57 entspricht, im Kreuzgegenstrom epoxidiert. Die Reaktoren und Abscheider besaßen jeweils ein Nutzvolumen von 100 I.

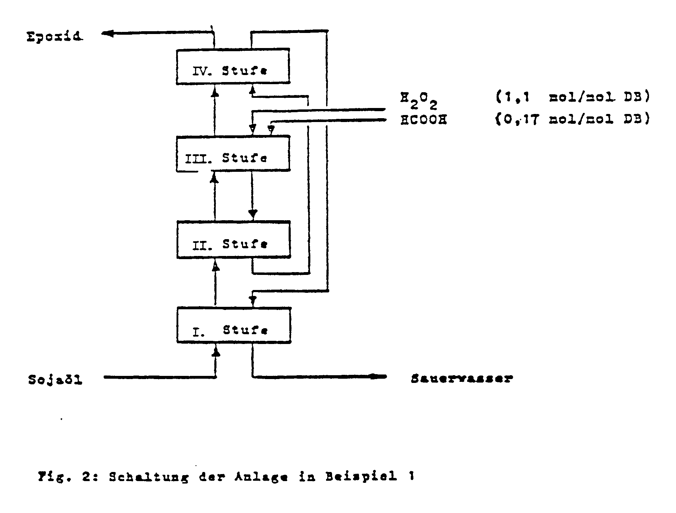

Die Führung der Stoffströme zeigt schematisch Fig. 2. In der dritten Stufe wurden 10,9 kg/h (1,1 mol/mol Doppelbindung (DB)) 70 %-iges Wasserstoffperoxid und 1,88 kg/h (0,17 mol/mol DB) 85 %-ige Ameisensäure zudosiert. Zur Stabilisierung der wässrigen Phase wurden dem $H_2O_2$-Einsatzstrom 0,1 kg/h Hydroxyethan-1,2-diphosphonsäure zugemischt. In jedem Abscheider wurden die Ölphase und die wässrige Phase separat gekühlt, so daß die Temperatur der Ölphase etwa 45°C und die der wässrigen Phase 20°C betrug. Die Reaktion wurde in allen Reaktoren bei 70°C durchgeführt. Man erhielt ein Sojaölepoxid mit einem Epoxidsauer stoffgehalt von 6,47 % und einer Jodzahl von 5,6. Das Sauerwasser verließ die Anlage mit einer Wasserstoffperoxidkonzentration von 6,2 Gew.-%.

## Beispiel 2

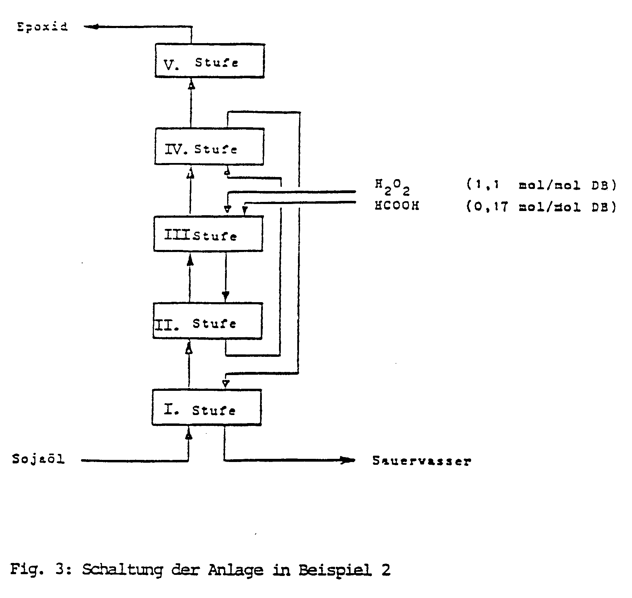

Beispiel 2 wurde durchgeführt wie Beispiel 1, jedoch mit einem zusätzlichen Nachreaktionsbehälter von 300 I Nutzvolumen, in dem die noch vorhandenen Doppelbindungen mit in der Olefinphase gelösten und dispergierten Bestandteilen der wässrigen Phase bei einer Tempertaur von 50°C weiter umgesetzt wurden (Fig. 3). Dadurch stieg der Expoxidwert auf 6,55, und die Jodzahl wurde auf 2,8 abgebaut.

## Beispiel 3

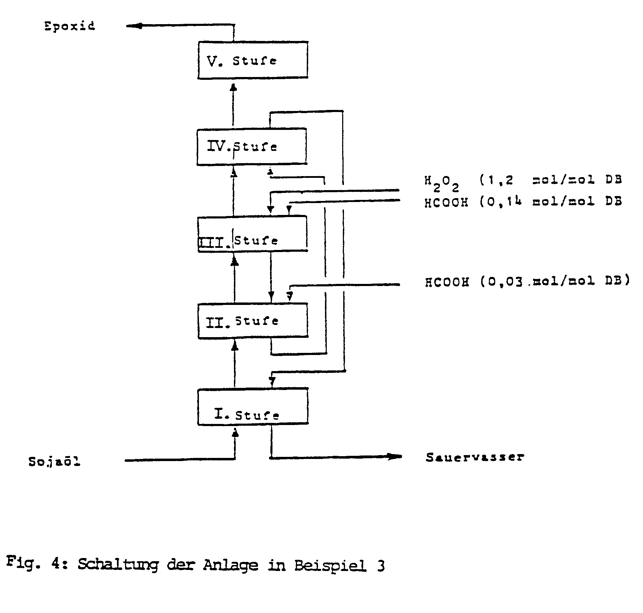

Gegenüber Beispiel 2 wurde der $H_2O_2$-Einsatz auf 11,9 kg/h (1,2 mol/mol DB) erhöht und die Ameisensäure genäß Fig. 4 auf die zweite und dritte Stufe aufgeteilt, so daß in der zweiten Stufe 0,03 mol/mol DB und in der dritten Stufe 0,14 mol/mol DB zudosiert wurden. Die Reaktortemperatur der vierten Stufe wurde auf 60°C gesenkt, alle übrigen Temperaturen entsprachen denen des Beispiels 2. Das erhaltene Epoxid besaß einen Epoxidwert von 6,62 und eine Jodzahl von 2,2.

## Beispiel 4 (Vergleichsbeispiel)

Dieses Beispiel wurde durchgeführt wie Beispiel 1, jedoch ohne Kühlung der Abscheider, so daß das Reaktionsgemisch bei etwa 60°C in den Abscheidern nachreagierte, wodurch sich die Phasentrennung deutlich verschlechterte. Man erhielt lediglich einen Epoxidwert von 5,88 und eine Jodzahl von 17,5.

## Beispiel 5 (Vergleichsbeispiel)

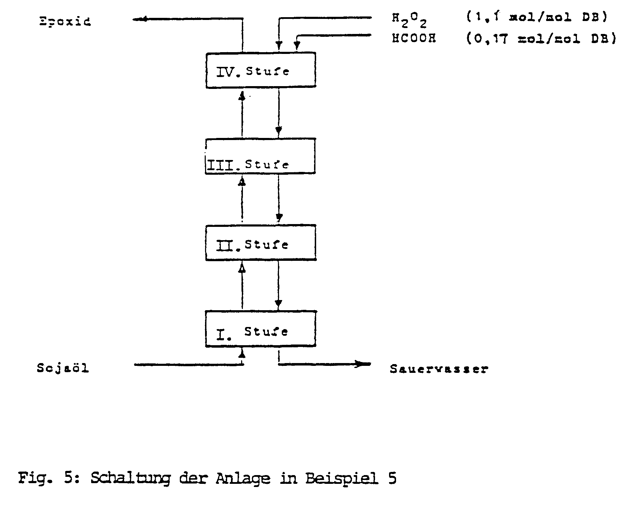

Die Stoffströme wurden im Gegenstrom entsprechend Fig. 5 geführt. Die Einsatzmengen sowie die Temperaturen der Reaktoren und Abscheider entsprachen denen des Beispiels 1. Obwohl das Wasserstoffperoxid der wässrigen Phase bis auf 5,0 Gew.-% abgebaut wurde, besaß das Epoxid lediglich einen Epoxidwert von 6,03, bei einer Jodzahl von 8,2, da ein Teil des hochkonzentrierten Sauerwassers in der vierten Stufe durch die Ölphase infolge unvollständiger Phasentrennung ausgeschleust wurde.

## Beispiel 6 (Vergleichsbeispiel)

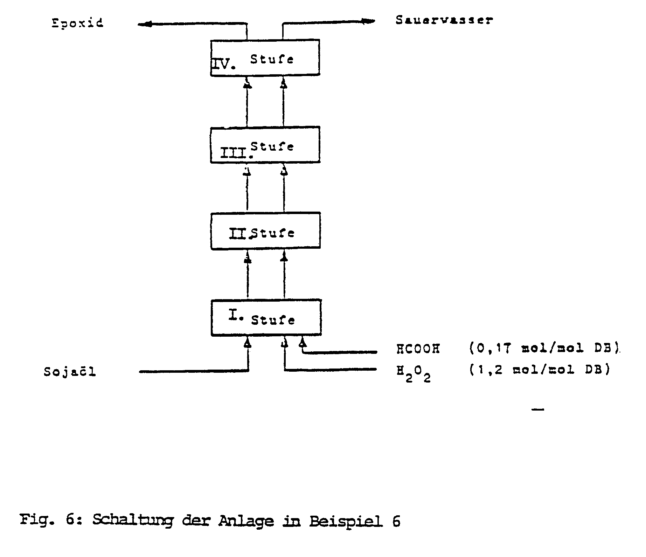

Das eingesetzte Sojaöl wurde in einer vierstufigen Gleichstromkaskade (keine Abscheider) epoxidiert (Fig. 6). Die Einsatzmengen betrugen 40 kg/h Sojaöl, 11,9 kg/h (1,2 mol/mol DB) 70 %-iges Wasserstoffperoxid, 1,88 kg/h (0,17 mol/mol DB) 85 %-ige Ameisensäure und 0,1 kg/h Hydroxyethan 1,2-diphosphonsäure. Alle Reaktionskomponenten wurden in der ersten Stufe zudosiert und durchliefen anschließend die Stufen II, III und IV. Die Reaktoren waren identisch mit denen des Beispiels 1. Die Reaktionstemperatur betrug in allen Reaktoren 70°C.

Mit diesen Reaktionsbedingungen erreichte man einen Epoxidwert von 6,37 und eine Jodzahl von 4,7. Das eingesetzte Wasserstoffperoxid wurde auf 14,8 Gew.% (bezogen auf die wässrige Phase der vierten Stufe) abgebaut.

**Beispiel 7**

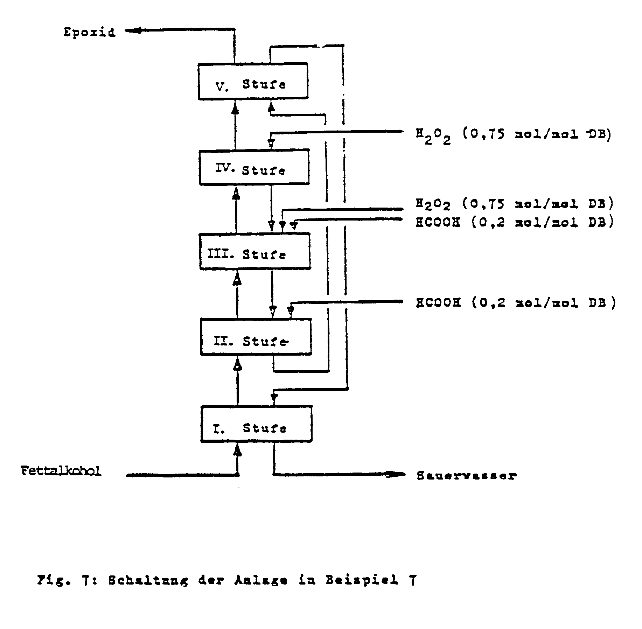

Ocenol[R] (ein ungesättigter Fettalkohol der Kettenlänge C16/18 mit einer Jodzahl von 88,3) wurde in einer fünfstufigen Mixer-Settler-Anlage entsprechend Fig. 7 epoxidiert. Die Reaktoren und Abscheider besaßen jeweils ein Nutzvolumen von 100 l. Die Einsatzmengen betrugen 60 kg/h des ungesättigten Fettalkohols, 15,2 kg/h (1,5 mol/mol DB) 70 %-iges Wasserstoffperoxid, 4,52 kg/h (0,4 mol/mol DB) 85 %-ige Ameisensäure und 0,14 kg/h Hydroxyethan-1,2-diphosphonsäure, die dem Wasserstoffperoxid zugemischt wurde. Wegen der Löslichkeit von Wasserstoffperoxid und Ameisensäure in der Alkoholphase wurde der Wasserstoffperoxideinsatz auf die dritte und vierte Stufe, der Ameisensäureeinsatz auf die Zweite und dritte Stufe aufgeteilt. Die Reaktion erfolgte bei 70°C in den Stufen I bis IV und bei 60°C in der Stufe V. In den Abscheidern wurde die Olefinphase auf 50°C und die wässrige Phase auf 20°C gekühlt.

Das auf diese Weise hergestellte Epoxid besaß einen Epoxidwert von 4,1 bei einer restlichen Jodzahl von 2,1, was einer Ausbeute von 77,8 % und einem Umsatz von 97,6 % entsprach.

**Beispiel 8**

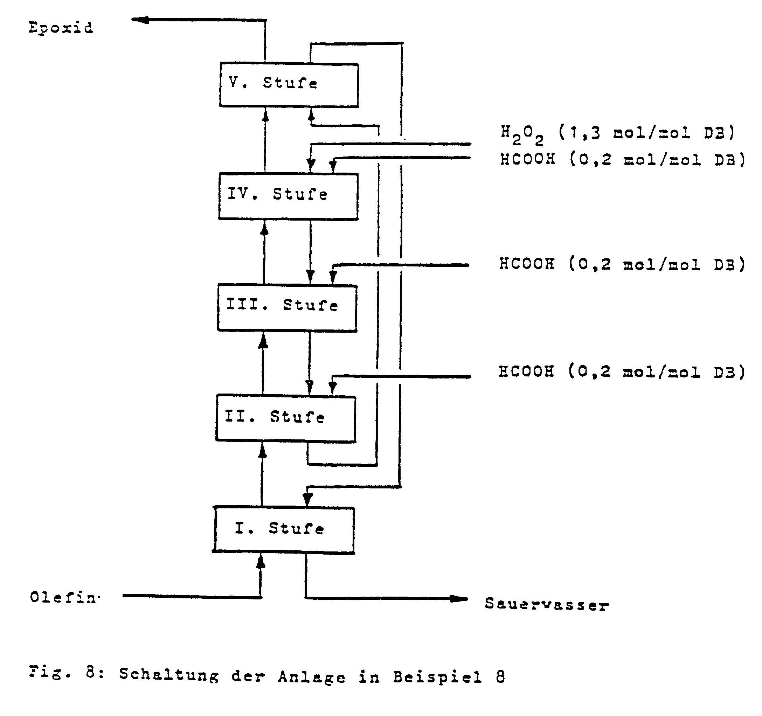

In einer fünfstufigen Mixer-Settler-Anlage wurde das α-Olefin Dodecen-1 kontinuierlich gemäß Fig. 8 epoxidiert. Ausgehend von einer Jodzahl von 148,2, könnte ein theoretischer Epoxidwert von 8,54 erreicht werden.

In der ersten Stufe wurden 12 kg/h Olefin und in der vierten Stufe 4,42 kg/h (1,3 mol/mol DB) 70 %-iges Wasserstoffperoxid zusammen mit 0,05 kg/h HEDP eingesetzt. Die 85 %-ige Ameisensäure wurde zu gleichen Teilen auf die Stufen II, III und IV aufgeteilt, um die Zersetzungsverluste gering zu halten. Insgesamt wurden 2,275 kg/h (0,6 mol/mol DB) Ameisensäure eingesetzt.

Die Reaktoren und Abscheider besaßen jeweils ein Nutzvolumen von 100 l. Die Temperatur betrug in allen Reaktoren 60°C; in den Abscheidern wurde die Temperatur der Olefinphase auf 40°C, die der Sauerwasserphase auf 20°C gehalten.

Unter diesen Reaktionsbediungungen wurde ein Epoxidwert von 4,58 und eine Jodzahl von 47,7 erreicht. Dies entsprach einer Ausbeute von 53,6 % bei einem Umsatz von 68,2 % Das Sauerwasser verließ die Anlage mit einer $H_2O_2$-Konzentration von 6,7 %.

**Patentansprüche**

1. Mehrstufiges Verfahren zur kontinuierlichen Epoxidation von Doppelbindungen end- und innenständiger Olefine mit mehr, als 12 C-Atomen, ungesättigter Fettsäuren und deren Ester sowie ungesättigter Alkohole mit 8 bis 18, vorzugsweise 18 C-Atomen im Alkylrest und Triglyceriden, vorzugsweise Sojaöl, unter Verwendung von in situ aus Wasserstoffperoxid und Ameisensäure gebildeter Perameisensäure, dadurch gekennzeichnet, daß man die Reaktanden Olefin und Wasserstoffperoxid/Ameisensäure in einer n-stufigen mindestens 3stufigen Reaktionskaskade, deren einzelne Stufen aus je einem Rührkesselreaktor und einem Phasenabscheider bestehen, bei Umgebungsdruck im Kreuzgegenstrom führt, wobei die Olefinphase in die erste Reaktionsstufe eingesetzt wird, das Wasserstoffperoxid und die Ameisensäure in getrennten Strömen der vorletzten Reaktionsstufe zudosiert und die Olefin- und die Wasserstoffperoxid/Ameisensäure-Phase (Sauerwasserphase) in jeder Reaktionsstufe nach Austritt aus dem Reaktor in einem Phasenabscheider voneinander getrennt werden und die olefinische Phase nacheinander die Reaktionsstufen I bis n durchläuft, während das Sauerwasser nach der Reaktionsstufe (n-1) die Reaktionsstufen (n-2) bis II im Gegenstrom zur Olefinphase und anschließend die Stufen n und I durchläuft und die Reaktionstemperatur in den Reaktoren 50 – 80°C, in den Phasenabscheidern jedoch in der wässrigen Phase 15 bis 40°C und in der olefinhaltigen Phase 30 bis 60°C beträgt.

2. Verfahren nach Anspruch 1, dadurch gekennzeichnet, daß der letzten Reaktionsstufe nach Abtrennung des Sauerwassers im Abscheider der letzten Reaktionsstufe ein weiterer Rührreaktor für die Olefin-Epoxidphase nachgeschaltet ist, der mit einer Temperatur von 30 – 70°C betrieben wird und in dem die in der Olefin-Epoxidphase gelösten und suspendierten Anteile an Wasserstoffperoxid und Ameisensäure bzw. Perameisensäure mit den noch nicht zur Reaktion gebrachten Doppelbindungen des Olefins reagieren können.

3. Verfahren nach Ansprüchen 1 und 2, dadurch gekennzeichnet, daß der Ameisensäurestrom zwischen der zweiten und (n-1)ten Reaktionsstufe verteilt zudosiert wird.

4. Verfahren nach Ansprüchen 1 – 3, dadurch gekennzeichnet, daß das Wasserstoffperoxid aufgeteilt auf mehrere Reaktionsstufen zwischen der zweiten und der (n-1)ten Reaktionsstufe zugeführt wird.

5. Verfahren nach Ansprüchen 1 – 4, dadurch gekennzeichnet, daß mit der Sauerwasserphase einer Reaktionsstufe ein Umwälzkreislauf zwischen Phasenabscheider und Reaktor der gleichen Reaktionsstufe betrieben wird, so daß der Anteil an Sauerwasserphase gegenüber dem des Olefins im Reaktor erhöht wird.

6. Verfahren nach Ansprüchen 1 – 5, dadurch gekennzeichnet, daß die Phasenabscheider der Reaktionsstufen mit einer abgestuften Kühlung betrieben werden, wobei die schwerere Sauerwasserphase auf 15 – 40°C, die leichtere Olefin-/Epoxidphase auf eine Temperatur von 30 – 60°C gekühlt wird.

7. Verfahren nach Ansprüchen 1 – 6, dadurch gekennzeichnet, daß die Reaktionstemperatur in den Reaktoren 60 – 70°C, in den Phasenabscheidern 40 – 50°C beträgt.

## Claims

1. A multistage process for the continuous epoxidation of double bonds of terminal ans internal olefins containing more than 12 C atoms, unsaturated fatty acids and esters thereof and unsaturated alcohols containing 8 to 18 and preferably 18 C atoms in the alkyl radical and triglycerides, preferably soybean oil, using performic acid formed in situ from hydrogen peroxide and formic acid, characterized in that the reactants olein and hydrogen peroxide/formic acid are moved in cross countercurrent through an nstage and at least 3-stage reaction cascade, of which the individual stages each consist of a stirred tank reactor and a phase separator, at ambient pressure, the olefin phase being introduced into the first reaction stage, the hydrogen peroxide and the formic acid being added in separate streams to the penultimate reaction stage and the olefin phase and the hydrogen peroxide/formic acid phase (acid water phase) in each reaction stage being separated from one another in a phase separator after leaving the reactor and the olefinic phase passing successively through reaction stages I to n while the acid water, after reaction stage (n-1), passes through reaction stages (n-2) to II in countercurrent to the olefin phase and the through stages n and I and the reaction temperature in the reactors being 50 to 80°C, but in the phase separators 15 to 40°C in the aqueous phase and 30 to 60°C in the olefincontaining phase.

2. A process as claimed in claim 1, characterised in that the last reaction stage after separation of the acid water in the saparator of the last reaction stage is followed by another stirred reactor for the olefin epoxide phase which is operated at a temperature of 30 to 70°C and in which the hydrogen peroxide and formic acid or performic acid dissolved and suspended in the olefin epoxide phase are able to react with the 80 yet unreacted double bonds of the olefin.

3. A process as claimed in claims 1 and 2, characterized in that the formic acid stream added is distributed between the second and the (n-1)th reaction stage.

4. A process as claimed in claims 1 to 3, characterized in that the hydrogen peroxide added is divided up among several reaction stages between the second the (n-1)th reaction stage.

5. A process as claimed in claims 1 to 4, characterized in that a circuit between the phase separator and reactor of the same reaction stage is established with the acid water phase of a reaction stage so that the proportion of acid water phase is increased over the proportion of olefin in the reactor.

6. A process as claimed in claims 1 to 5, characterized in that the phase separators of the reaction stages are operated with graduated cooling, the heavier acid water phase being cooled to 15 – 40°C and the light olefin/epoxide phase being cooled to a temperature of 30 – 60°C.

7. A process as claimed in claims 1 to 6, characterized in that the reaction temperature in the reactors is 60 – 70°C and in the phase separators 40 – 50°C.

8. A process as claimed in claims 1 to 7, characterized in that the separation layer between olefin/epoxide and acid water phase in the separators is adjusted so that the ratio by volume of acid water phase to olefin/epoxide phase in the separators is lower than or at most equal to that of the added streams so that the average residence time of the acid water phase is below or at most equal to that of the epoxide phase.

9. A process as claimed in claims 1 to a, characterized in that, to prevent convection currents, the separators optionally contain a glass or ceramic packing.

## Revendications

1. Procédé à plusieurs étages destiné à l'époxydation continue de double liaisons terminales et non terminales d'oléfines contenant plus de 12 atomes de carbone, d'acides gras insaturés et de leurs esters, de même que d'alcools insaturés contenant 8 à 18, de préférence 18 atomes de carbone dans le radical alkyle, et de triglycérides, de préférence, d'huile de soja, en utilisant de l'acide performique formé in situ à partir de peroxyde d'hydrogène et d'acide formique, ce procédé étant *caractérisé en ce que* l'on envoie, sous pression ambiante et à contre-courants croisés, l'oléfine et le peroxyde d'hydrogène/acide formique en réaction, dans une cascade réactionnelle à n étages, à au moins 3 étages, de préférence, à 4 étages, chacun de ces étages individuels comprenant un réacteur de chaudière à agitateur et un séparateur de phases, la phase oléfinique étant introduite au premier étage réactionnel, tandis que l'on procédé à une addition dosée du peroxyde d'hydrogène et de l'acide formique, en courants séparés, à l'avant dernier étage, la phase oléfinique et la phase peroxyde d'hydrogène/acide formique (phase d'eau acide) étant séparées l'une de l'autre, dans un séparateur de phases, au niveau de chaque étage réactionnel, après sortie hors du réacteur, tandis que la phase oléfinique traverse successivement les étages réactionnels 1 à n, l'eau acide traversant après l'étage réactionnel (n-1) successivement les étages réactionnels (n-2) à II, à contrecourant par rapport à la phase oléfinique, tandis qu'elle traverse ensuite les étages n et I, la température réactionnelle régnant dans les réacteurs étant de 50 - 80°C, tandis que dans les séparateurs de phases toutefois, elle est de 15 à 40°C dans la phase aqueuse et de 30 à 60°C, dans la phase oléfinique.

2. Procédé selon la revendication 1, *caractérisé en ce que*, à la suite du dernier étage réactionnel, après à la séparation de l'eau acide intervenue dans le séparateur du dernier étage réactionnel, on intercale un réacteur supplémentaire à agitateur pour la phase oléfine-époxyde, ce réacteur fonctionnant à une température de 30 − 70°C, tandis que les fractions dissoutes et en suspension du peroxyde d'hydrogène et de l'acide formique ou de l'acide performique, ces fractions se trouvant en phase oléfine-époxyde, peuvent v être mises en réaction avec les doubles liaisons de l'oléfine n'ayant pas encore réagi.

3. Procédé selon les revendications 1 et 2, *caractérisé en ce que* l'on procédé à l'addition dosée du courant d'acide formique en le répartissant entre le deuxième et l'étage (n-1) réactionnel.

4. Procédé selon les revendications 1 − 3, *caractérisé en ce que* l'on achemie le peroxyde d'hydrogène réparti sur plusieurs étages réactionnels, entre le deuxième et l'étage (n-1) réactionnel.

5. Procédé selon les revendications 1 − 4, *caractérisé en ce qu'*avec la phase d'eau acide d'un étage réactionnel, on effectue un recyclage entre le séparateur de phases et le réacteur du même étage réactionnel, si bien que la fraction en phase d'eau acide augmente dans le réacteur par rapport à celle de l'oléfine.

6. Procédé selon les revendications 1 − 5, *caractérisé en ce que* l'on fait fonctionner les séparateurs de phases de étages réactionnels avec un refroidissement gradué, la phase d'eau acide plus lourde étant refroidie à une température de 15 - 40°C, tandis que la phase oléfine-époxyde plus légère est refroidie à 30 − 60°C.

7. Procédé selon les revendications 1 − 6, *caractérisé en ce que* la température réactionnelle régnant dans les réacteurs est de 60 - 70°C, tandis que celle régnant dans les séparateurs de phases est de 40 - 50°C.

8. Procédé selon les revendications 1 − 7, *caractérisé en ce que* l'on règle la position de la couche de séparation entre la phase oléfine-époxyde et la phase d'eau acide dans les séparateurs de telle façon que le rapport des volumes entre la phase d'eau acide et la phase oléfine-époxyde dans les séparateurs soit inférieur ou tout au plus égal à celui des courants de dosage, si bien que le temps moyen de séjour de la phase d'eau acide soit inférieur ou tout au plus égal à celui de la phase époxyde.

9. Procédé selon les revendications 1 − 8, *caractérisé en ce que* les séparateurs, en vue d'empêcher les courants de convection, sont éventuellement munis de matières de remplissage constituées de céramique ou de verre.

FIG. 1  Schematische Darstellung des Kreuzgegenstromverfahrens zur kontinuierlichen Epoxidation von Sojaöl

Epoxid

IV. Stufe

$H_2O_2$     (1,1  mol/mol DB)

HCOOH     (0,17 mol/mol DB)

III. Stufe

II. Stufe

I. Stufe

Sojaöl                                    Sauerwasser

Fig. 2: Schaltung der Anlage in Beispiel 1

Epoxid

V. Stufe

IV. Stufe

$H_2O_2$          (1,1  mol/mol DB)

HCOOH          (0,17 mol/mol DB)

III Stufe

II. Stufe

I. Stufe

Sojaöl                                          Sauervasser

Fig. 3: Schaltung der Anlage in Beispiel 2

Epoxid

```
                    ┌──────────────┐
                    │  V. Stufe    │
                    └──────────────┘
                           ↑
                    ┌──────────────┐
                    │  IV. Stufe   │
                    └──────────────┘
                                          H₂O₂  (1,2  mol/mol DB
                                          HCOOH (0,14 mol/mol DB
                    ┌──────────────┐
                    │ III. Stufe   │
                    └──────────────┘
                                          HCOOH (0,03 mol/mol DB)
                    ┌──────────────┐
                    │  II. Stufe   │
                    └──────────────┘

                    ┌──────────────┐
                    │  I. Stufe    │
                    └──────────────┘
                           ↑
Sojaöl ──────────────────────┘         ────────→  Sauerwasser
```

Fig. 4: Schaltung der Anlage in Beispiel 3

Epoxid

H$_2$O$_2$   (1,1 mol/mol DB)
HCOOH  (0,17 mol/mol DB)

IV. Stufe

III. Stufe

II. Stufe

I. Stufe

Scjaöl                              Sauervasser

Fig. 5: Schaltung der Anlage in Beispiel 5

Epoxid ←————————————————→ Sauervasser

```
          ┌──────────────┐
          │ IV.  Stufe   │
          └──────────────┘
             ↑       ↑
          ┌──────────────┐
          │ III. Stufe   │
          └──────────────┘
             ↑       ↑
          ┌──────────────┐
          │ II Stufe     │
          └──────────────┘
             ↑       ↑
          ┌──────────────┐
          │ I. Stufe     │
          └──────────────┘
           ↑    ↑   ↑
                        ←———— HCOOH   (0,17 mol/mol DB)
Sojaöl ———————————————— H₂O₂    (1,2 mol/mol DB)
```

HCOOH   (0,17 mol/mol DB)

$H_2O_2$    (1,2 mol/mol DB)

Fig. 6: Schaltung der Anlage in Beispiel 6

Fig. 7: Schaltung der Anlage in Beispiel 7

Fig. 8: Schaltung der Anlage in Beispiel 8